# EUROPEAN PATENT APPLICATION

(11) **EP 2 241 311 A1**
(43) Date of publication of application: **20.10.2010**
(21) Application number: 08790105.4
(22) Date of filing: 30.06.2008
(51) Int. Cl.: A61K 9/32, A61K 47/02, A61K 47/04, A61K 47/12, A61K 47/14, A61K 47/32, A61K 47/36, A61K 47/38

(54) **TIMED-RELEASE PHARMACEUTICAL PREPARATION**

(30) Priority: 15.02.2008 JP 2008035056
(71) Applicant: SSP Co., Ltd., Tokyo 103-8481 (JP)
(72) Inventor: KANBE, Hideyoshi, Narita-shi Chiba 286-8511 (JP)
(74) Representative: Hartz, Nikolai
(86) International application number: PCT/JP2008/001706
(87) International publication number: WO 2009/101657

(57) **Abstract**

The timed-release pharmaceutical preparation of the invention is **characterized by** having a core containing a drug and a water-swellable substance, and a film containing a water-insoluble polymer, a plasticizer, and a water-insoluble filler, the core being coated with the film.

With the timed-release pharmaceutical preparation of the present invention, the drug-release-initiation time and the drug release rate after the initiation of drug release can be controlled as desired.

## Description

### Technical Field

The present invention relates to a timed-release pharmaceutical preparation, with which the period of time required until initiation of release of a drug from that preparation (hereinafter may be referred to as "drug-release-initiation time") and the drug release rate after the initiation of drug release can be controlled as desired.

### Background Art

Most drugs which have been perorally administered to a subject in need thereof are typically absorbed by the small intestine which they pass the stomach, duodenum, small intestine, and large intestine. For a certain disease of the large intestine such as ulcerative colitis, however, a drug must be correctly delivered to a downstream portion of the small intestine and to the large intestine. In the case where particular therapeutic drugs such as a peptide-type drug-readily undergoing chemical or enzymatic degradation in the gastrointestinal tract-and a drug which is absorbed by specific sites are employed through, for example, peroral administration, there is a keen demand for selective delivery of such drugs to the large intestine.

In the gastrointestinal tract, generally, the pH in the stomach is 1.8 to 4.5, and that in the small intestine and large intestine is 6.5 to 7.5. The stomach residence time of a drug preparation is 0.5 to 10 hours, but the time varies considerably among subjects and is thought to greatly depend on the size of the preparation and the meal taken therewith. In contrast, in the small intestine, the time for the passage of a drug preparation does not vary considerably, and the passage time is generally 3 ± 1 hours.

In order to correctly deliver a drug to a downstream portion of the small intestine and the large intestine, particularly, a timed-release pharmaceutical preparation which exhibits a release property not depending on the residence time in the stomach or the pH in the gastrointestinal tract is required. In other words, there is required a timed-release pharmaceutical preparation which does not release a drug in the acidic and neutral pH regions and which can release the drug in the alkaline pH region after passage of a certain period of time.

One conventional technique for releasing a drug after it has passed through the stomach uses an enteric preparation. This preparation is in the form of tablet, granules, etc., coated with an organic polymeric substance which is generally insoluble in gastric juice and soluble in intestinal juice. Thus, since the enteric preparation does not release a drug in the stomach and rapidly releases the drug in the small intestine, the drug cannot be delivered to a downstream portion of the small intestine and the large intestine.

Some peroral administration systems are known to release a drug specifically to a downstream portion of the gastrointestinal tract, such as a downstream portion of the small intestine and the large intestine. One example thereof is a timed-release pharmaceutical preparation which controls the release initiation time based on the gastrointestinal tract passage time of the drug.

One example of the timed-release pharmaceutical preparation exhibiting a certain period of lag time is a pharmaceutical preparation containing core particles and, affixed to the surfaces thereto, a water-swellable substance and a drug. The resultant particles are coated with a film formed of a mixture of ethyl cellulose and talc, wherein the film is broken by swelling of the water-swellable substance, to thereby release the drug (Patent Documents 1 and 2). There have also been known, for example, a pharmaceutical preparation having a film for attaining lag time until the drug release, which film is formed from a water-repellent salt such as a fatty acid metal salt (e.g., magnesium stearate or calcium stearate) and an acrylic polymer (Patent Document 3), and a pharmaceutical preparation employing interaction between Eudragit RS (product of Degussa Japan) and organic acid (Patent Document 4).
However, since the drug release of these pharmaceutical preparations depends on the residence time in the stomach, the drug release site is thought to be difficult to determine.

Since the small intestine passage time of a drug is virtually constant among subjects, among the delivery systems employing the gastrointestinal tract passage time of a drug, some delivery systems employing the small intestine passage time of a drug have been developed so as to prevent variation in stomach residence time. That is, such a system is a controlled-release-type pharmaceutical preparation which also has an enteric film function.

Examples of such a pharmaceutical preparation include a timed-release pharmaceutical preparation employing interaction between Eudragit RS (product of Degussa Japan) and organic acid, the preparation being coated with an enteric polymer (Patent Document 5); a granular agent having a 5-layered structure including a core containing an acidic substance, coated sequentially with a water-insoluble polymer, a drug layer, a low-pH-soluble polymer, and an enteric polymer (Patent Document 6); a core/shell tablet having a core containing a drug and an acidic substance, the core being coated with a compressed layer of low-pH-soluble polymer and a compressed layer of an enteric polymer (Patent Document 7); and a capsule agent having a capsule containing an acidic substance, the capsule being coated with a water-insoluble polymer, a low-pH-soluble polymer, and an enteric polymer (Patent Document 8).

Patent Document 1: JP-B-1995-72130
Patent Document 2: JP-A-1995-196477
Patent Document 3: Japanese Patent No. 2558396
Patent Document 4: JP-B-1994-74206
Patent Document 5: JP-A-1995-2650
Patent Document 6: JP-A-1995-10745
Patent Document 7: JP-A-1995-223970
Patent Document 8: JP-A-1997-87169

### Disclosure of the Invention

### Problems to be Solved by the Invention

Each of the aforementioned pharmaceutical preparations does not release a corresponding drug when it is in the stomach and releases the drug a certain period of time after the preparation has been transferred from the stomach to the small intestine. That is, the preparation can release the drug at a specific site a certain distance away from the stomach; e.g., the large intestine. The aforementioned pharmaceutical preparations may be produced as granules having a multilayer structure including spherical core granules made of, for example, lactose (e.g., Nonpareil 101 (product of Freund Corporation)), each granule being coated with an acidic substance layer, a drug layer, a release-controlling layer, an enteric layer, and other layers. According to these conventional pharmaceutical preparations, a drug delivery system having, in combination, a function of enteric film and a drug-release-time-controlling function can be established. However, such pharmaceutical preparations have a complex structure and are produced through a long and cumbersome process, requiring high-level drug preparation techniques. Thus, there is a keen demand for a timed-release pharmaceutical preparation which can be produced through a simple production method without requiring high-level drug preparation techniques and which immediately releases the drug at a specific site of the large intestine or small intestine a certain lag time after the preparation has passed through the stomach.
Thus, an object of the present invention is to provide a timed-release pharmaceutical preparation, with which the drug-release-initiation time and the drug release rate after the initiation of drug release can be controlled as desired.

### Means for Solving the Problems

The present inventors have carried out extensive studies in order to develop a timed-release pharmaceutical preparation, with which the drug-release-initiation time and the drug release rate after the initiation of drug release can be controlled as desired, and have found that the aforementioned object can be attained by a pharmaceutical preparation having a core containing a drug and a water-swellable substance, and a film containing a water-insoluble polymer, a plasticizer, and a water-insoluble filler, the core being coated with the film. The present inventors have carried out further extensive studies, and have found that when a (meth)acrylic water-insoluble polymer is used as the water-insoluble polymer, there can be provided a timed-release pharmaceutical preparation which does not release a drug in the acidic and neutral pH regions and rapidly releases the drug in the alkaline pH region after passage of a certain period of lag time.

Accordingly, the present invention provides a timed-release pharmaceutical preparation characterized by having a core containing a drug and a water-swellable substance, and a film containing a water-insoluble polymer, a plasticizer, and a water-insoluble filler, the core being coated with the film.

### Effects of the Invention

According to the present invention, through coating a core containing a drug and a water-swellable substance with only one layer of a film containing a water-insoluble polymer, a plasticizer, and a water-insoluble filler and modifying the composition of the film, the coating amount, and the water-swellable substance content of the core, there can be provided a timed-release pharmaceutical preparation, with which the drug-release-initiation time and the drug release rate after the initiation of drug release can be controlled as desired. In addition, the preparation of the present invention never releases a drug in a low pH region (e.g., in the stomach) and can break the film only in a virtually neutral pH region (e.g., in the small intestine and large intestine), when a (meth)acrylic water-insoluble polymer is employed as the water-insoluble polymer. Thus, a certain period of time when the preparation does not release a drug in the alkaline region (i.e., lag time) is realized, to thereby provide a pH-dependent timed-release pharmaceutical preparation which can rapidly release the drug after the lag time.

### Brief Description of the Drawings

[Fig. 1] Fig. 1 is a graph showing calculation, from an elution curve, of the lag time (the period of time during which the preparation does not release a drug), T_{80%} (the point in time at which the elution amount reaches 80%) and T_{80%}-(lag time) (drug-releasability of the timed-release pharmaceutical preparation).
[Fig. 2] Fig. 2 is a graph showing elution curves of the pharmaceutical preparation produced in Example 1.
[Fig. 3] Fig. 3 is a graph showing elution curves of the pharmaceutical preparation produced in Example 1.
[Fig. 4] Fig. 4 is a graph showing elution curves of the pharmaceutical preparation produced in Example 2.
[Fig. 5] Fig. 5 is a graph showing elution curves of the pharmaceutical preparation produced in Example 2.
[Fig. 6] Fig. 6 is a graph showing elution curves of the pharmaceutical preparation produced in Example 3.
[Fig. 7] Fig. 7 is a graph showing elution curves of the pharmaceutical preparation produced in Example 3.
[Fig. 8] Fig. 8 is a graph showing elution curves of the pharmaceutical preparation produced in Example 4.
[Fig. 9] Fig. 9 is a graph showing elution curves of the pharmaceutical preparation produced in Example 4.
[Fig. 10] Fig. 10 is a graph showing elution curves of the pharmaceutical preparation produced in Example 5.
[Fig. 11] Fig. 11 is a graph showing elution curves of the pharmaceutical preparation produced in Example 5.
[Fig. 12] Fig. 12 is a graph showing elution curves of the pharmaceutical preparation produced in Example 6.
[Fig. 13] Fig. 13 is a graph showing elution curves of the pharmaceutical preparation produced in Example 6.
[Fig. 14] Fig. 14 is a graph showing elution curves of the pharmaceutical preparation produced in Example 7.
[Fig. 15] Fig. 15 is a graph showing elution curves of the pharmaceutical preparation produced in Example 7.
[Fig. 16] Fig. 16 is a graph showing elution curves of the pharmaceutical preparation produced in Example 8.
[Fig. 17] Fig. 17 is a graph showing elution curves of the pharmaceutical preparation produced in Example 8.
[Fig. 18] Fig. 18 is a graph showing elution curves of the pharmaceutical preparation produced in Example 9.
[Fig. 19] Fig. 19 is a graph showing elution curves of the pharmaceutical preparation produced in Example 9.
[Fig. 20] Fig. 20 is a graph showing elution curves of the pharmaceutical preparation produced in Example 10.
[Fig. 21] Fig. 21 is a graph showing elution curves of the pharmaceutical preparation produced in Example 10.
[Fig. 22] Fig. 22 is a graph showing elution curves of the pharmaceutical preparation produced in Example 11.
[Fig. 23] Fig. 23 is a graph showing elution curves of the pharmaceutical preparation produced in Example 11.
[Fig. 24] Fig. 24 is a graph showing elution curves of the pharmaceutical preparation produced in Example 12.
[Fig. 25] Fig. 25 is a graph showing elution curves of the pharmaceutical preparation produced in Example 12.
[Fig. 26] Fig. 26 is a graph showing elution curves of the pharmaceutical preparation produced in Example 13.
[Fig. 27] Fig. 27 is a graph showing elution curves of the pharmaceutical preparation produced in Example 14.
[Fig. 28] Fig. 28 is a graph showing elution curves of the pharmaceutical preparation produced in Example 15.
[Fig. 29] Fig. 29 is a graph showing elution curves of the pharmaceutical preparation produced in Example 16.
[Fig. 30] Fig. 30 is a graph showing elution curves of the pharmaceutical preparation produced in Example 17.
[Fig. 31] Fig. 31 is a graph showing elution curves of the pharmaceutical preparation produced in Example 18.

### Best Modes for Carrying Out the Invention

One characteristic feature of the timed-release pharmaceutical preparation of the present invention resides in that the preparation has a double-layer structure including a core and a film covering the core surface, wherein the core contains a drug and a water-swellable substance, and the film contains a water-insoluble polymer, a plasticizer, and a water-insoluble filler.

No particular limitation is imposed on the drug employed in the present invention, so long as the drug can be perorally administered. Examples of the drug include a chemotherapeutic agent, an antibiotic, a respiratory stimulant, an antitussive/expectorant, an anti-malignant-tumor agent, a drug for autonomic nervous system, a psyconeurotic drug, a local anesthesia, a muscle relaxant, a drug for the digestive tract, an antihistamine agent, an intoxication treatment agent, a hypnotic/sedative, an antipyretic analgesic/antiinflammatory agent, a cardiotonic agent, an antiarrhythmic agent, a diuretic, a vasodilator, an antilipemic agent, an analeptic nutrient, an anticoagulant, a drug for the liver, a hypoglycemic agent, an antihypertensive, a colitis-treating agent, a peptide, and a protein. Of these, a colitis-treating agent, which must be sufficiently absorbed in the large intestine serving as a disease site, and a peptide and a protein, which tend to be degraded in the stomach or the like, are preferred.

The drug content of the core may be appropriately determined in accordance with the purpose of the preparation. From the viewpoints of the lag time and drug releasability after the lag time, the drug content of the composition forming the core is preferably 85 mass% or less, more preferably 70 mass% or less, particularly preferably 60 mass% or less. The lower limit of the drug content is preferably 3 mass%, particularly preferably 5 mass%, from the viewpoint of pharmacological effects.

Examples of the water-swellable substance forming the core include low-substituted hydroxypropyl cellulose, carmellose or a salt thereof, croscarmellose sodium, carboxymethylstarch sodium, crospolyvinylpyrrolidone, crystalline cellulose, and crystalline cellulose/carmellose sodium. Of these, low-substituted hydroxypropyl cellulose is particularly preferred. The low-substituted hydroxypropyl cellulose preferably has a hydroxypropoxyl group content of about 7.0 to about 16.0 mass%, more preferably about 10 to about 12.9 mass%, and a mean particle size of 30 µm or less, particularly preferably 20 µm or less.

The aforementioned water-swellable substances may be used singly or in combination of two or more species. The water-swellable substance content of the core is 15 mass% or more, more preferably 30 mass% or more, particularly preferably 40 mass% or more. From the viewpoint of the drug content, the upper limit of the water-swellable substance content is preferably 97 mass%, particularly preferably 95 mass%.

The core may further contain a variety of additives which may generally be employed in the art; such as a filler, a binder, a lubricant, a coagulation inhibitor, and a solubilizer for pharmaceutical compounds. Examples of the filler include saccharides such as sucrose, lactose, mannitol, and glucose; starch; crystalline cellulose; calcium phosphate; and calcium sulfate. Examples of the binder include poly(vinyl alcohol), poly(acrylic acid), poly(methacrylic acid), polyvinylpyrrolidone, glucose, sucrose, lactose, maltose, dextrin, sorbitol, mannitol, hydroxyethyl cellulose, hydroxypropylmethyl cellulose, hydroxypropyl cellulose, Macrogols, arabic gum, gelatin, agar, and starch. Examples of the lubricant and coagulation inhibitor include talc, magnesium stearate, calcium stearate, colloidal silica, stearic acid, waxes, hardened oil, polyethylene glycol, and sodium benzoate. Examples of the solubilizer for pharmaceutical compounds include organic acids such as fumaric acid, succinic acid, malic acid, and adipic acid. The amounts of these additives incorporated into the core may be appropriately determined in accordance with the type of the drug and other factors.

No particular limitation is imposed.on the water-insoluble polymer which forms the film, and any polymer may be appropriately employed, so long as the polymer is a water-insoluble polymeric compound. Among such polymers, a (meth)acrylic water-insoluble polymer is preferred, with ethyl acrylate-methyl methacrylate-trimethylammoniumethyl methacrylate chloride terpolymer being particularly preferred. The proportions (by mass) of ethyl acrylate, methyl methacrylate, and trimethylammoniumethyl methacrylate chloride, which form the terpolymer, are preferably 1:2:0.1 to 1:2:0.2. Examples of the commercial product thereof include Eudragit RS (e.g., RSPO, RS100, or RS30D) and Eudragit RL (e.g., RLPO, RL100, or RL30D) (these products available from Degussa Japan). Eudragit RS has a trimethylammonium chloride group content of 4.48 to 6.77 mass%, and Eudragit RL has a trimethylammonium chloride group content of 8.85 to 11.96 mass%.
These water-insoluble polymers may be used singly or in combination of two or more species. For example, two terpolymers having different trimethylammonium chloride group contents may be used. In the case where Eudragit RS and Eudragit RL are used in combination, the ratio (RS:RL, by mass) is preferably 9.5:0.5 to 5:5, more preferably 9.75:0.25 to 5:5, particularly preferably 7:3 to 5:5.

Preferably, the film has a water-insoluble polymer content (solid content) of 30 mass% or more, more preferably 40 mass% or more, particularly preferably 45 mass% or more. The upper limit of the polymer content is preferably 95 mass%, particularly preferably 90 mass%, from the viewpoint of coatability.

Examples of the plasticizer include triethyl citrate, glycerin fatty acid esters, and triacetin. Of these, triethyl citrate and glycerin fatty acid esters are preferred, with triethyl citrate being particularly preferred. The fatty acid forming the glycerin fatty acid ester may be saturated or unsaturated and preferably has 4 to 36 carbon atoms, particularly preferably 12 to 22 carbon atoms.
Preferably, the film has a plasticizer content (with respect to the total mass of the water-insoluble polymer) (solid content) of 2 mass% or more, more preferably 4 mass% or more, still more preferably 6 mass% or more, yet more preferably 7 mass% or more, particularly preferably 7.5 mass% or more, more particularly preferably 8 mass% or more. The upper limit thereof is preferably 50 mass%, more preferably 40 mass%, particularly preferably 35 mass%. Under the above conditions of the plasticizer content, there can be provided a pH-dependent timed-release pharmaceutical preparation which never releases a drug in the low-pH region and rapidly releases the drug in the alkaline region after a certain period of lag time.

Examples of the water-insoluble filler include talc, magnesium stearate, calcium stearate, kaolin, titanium oxide, magnesium oxide, calcium carbonate, magnesium carbonate, calcium phosphate, calcium sulfate, and aluminum hydroxide dry gel. Of these, talc, kaolin, and titanium oxide are preferred, with talc (mean particle size: 10 to 40 µm) being more preferred and pulverized talc (mean particle size: 1 to 10 µm) being particularly preferred. Such talc products are commercially available as, for example, Victoria Light SK-C (mean particle size: 3.45 µm, product of Katsuyama Kogyosho) or Victoria Light SK-BB (mean particle size: 4.6 µm, product of Katsuyama Kogyosho).

Preferably, the film has a water-insoluble filler content (with respect to the total mass of the film) of 15 mass% or more, more preferably 25 mass% or more, particularly preferably 30 mass%. The upper limit thereof is preferably 80 mass%, more preferably 70 mass%, particularly preferably 60 mass%.

The film may further contain additives such as wax, stearic acid, a masking agent, a colorant, a perfume, a lubricant, and an anti-coagulant. The amounts of these additives incorporated into the film may be appropriately determined in accordance with the type of the drug and other factors.

One preferred embodiment of production of the pharmaceutical preparation of the present invention will next be described.
Firstly, a drug and a water-swellable substance are mixed with an optional additive(s), and the mixture is mixed by means of an agitation mixer (e.g., Vertical Granulator (product of Powrex Corporation)). The product is kneaded with purified water or aqueous alcohol, to thereby form a swelling mixture.
Examples of the alcohol contained in the aqueous alcohol include ethyl alcohol, methyl alcohol, and isopropyl alcohol, which may be employed in pharmaceutical products or for producing the same. The alcohol concentration is preferably 50 mass% or less, particularly preferably 30 mass% or less. The lower limit thereof is preferably 5 mass%, particularly preferably 10 mass%. Water or aqueous alcohol is employed as a kneading solvent for wet granulation so as to render the water-swellable substance to a swelled state. The amount by mass of the kneading solvent used with respect to the amount by mass of the water-swellable substance is preferably 2 to 5 times, particularly preferably 2 to 3 times. To the kneading solvent composed of water or aqueous alcohol, in accordance with needs, there may be added additives that may generally be employed in pharmaceutical products, such as a sweetener, a sugar (e.g., fine sucrose), a sugar alcohol (e.g., D-mannitol), and a polymer dissolved or dispersed in water.

The core of the present invention is preferably produced through wet granulation. No particular limitation is imposed on the specific method of wet granulation, so long as the method is agitation granulation, fluidized bed granulation, or extrusion granulation. Among them, extrusion granulation is preferred. Particularly preferably, the granules obtained through extrusion granulation are rendered to be spherical by means of Marumerizer. Specifically, a kneaded material in a swelling state is subjected to extrusion granulation by means of an extrusion granulator (e.g., Twin Dome Granulator, product of Fuji Paudal Co., Ltd., equipped with a screen having a mesh size of 0.3 to 1.0 mm). Thereafter, the thus-obtained granules are rendered to be spherical by means of Marumerizer (product of Fuji Paudal Co., Ltd.) and dried by means of a box-type drier or a fluidized-bed drier. Subsequently, the thus-produced core granules containing a drug and a water-swellable substance are coated with a coating liquid containing a water-insoluble polymer, a plasticizer, and a water-insoluble filler, whereby the pharmaceutical preparation of the present invention can be produced. If required, the aforementioned additives and other additives may be added to the preparation. Examples of the solvent for dispersing the coating base therein include water; alcohols such as methanol and ethanol; ketones such as acetone; halo-hydrocarbons such as methylene chloride and chloroform; and mixtures thereof. Of these, water, alcohols, and mixtures thereof are preferred, with ethanol and a mixture of ethanol and water being particularly preferred. The alcohol concentration of the aqueous alcohol solution may be adjusted as desired. However, through adjusting the alcohol concentration to lower than 80 mass%, particularly 20 to 60 mass%, there can be produced a timed-release pharmaceutical preparation which has a lag time of 5 minutes or shorter, 10 minutes or shorter, or 15 minutes or shorter and which releases 80 mass% or more of the drug within 12 minutes, 15 minutes, or 20 minutes after passage of the lag time. When the drug gives an unpleasant bitter taste or the like, such a taste of the drug during taking thereof may be masked to provide a masked timed-release pharmaceutical preparation.

Any customary methods employed in drug preparation may be applied to coating the core with a film, and examples include fluidized-bed coating, pan-coating, and tumbling fluidized-bed coating. When fluidized-bed coating is employed, in one specific procedure, a dispersion of the coating base is sprayed at an appropriate speed through the nozzles of a spray gun onto the core substance, while the core substance is fluidized by air pressure in the coating apparatus.

No particular limitation is imposed on the coating base concentration of the coating liquid, but the concentration is preferably 5 to 30 mass%, from the viewpoints of film formation performance, workability, etc. The thus-produced pharmaceutical preparation of the present invention may be administered in the aforementioned drug form, or encapsulated for administration. Further, the preparation may be formed into tablets or, if required, into coated (e.g., sugar-coated) tablets.

The coating amount of the film is preferably 10 mass% or more with respect to the total mass of the core, more preferably 30 mass% or more, particularly preferably 50 mass% or more, from the viewpoints of lag time and drug releasability after passage of lag time. The upper limit of the coating amount is preferably 300 mass%, particularly preferably 250 mass%.

As described above, according to the present invention, the core containing a drug and a water-swellable substance is preferably produced through extrusion granulation of raw materials in a swelling state and rendering the granules to be spherical by means of Marumerizer. Therefore, core particles having a particle size smaller than the screen mesh size can be produced, since the water-swellable substance shrinks during drying. Through employment of this procedure, spherical core particles containing a drug and a water-swellable substance can be produced without requiring high-level drug preparation techniques, as compared with a conventional method in which a drug and a water-swellable substance in powder form are applied to core particles (Nonpareil, mean particle size: 840 to 350 µm) by means of a conventional centrifugal tumbling granulator (e.g., CF Granulator, product of Freund Corporation), while an aqueous solution of a binder is sprayed to the particles. In addition, when the method employing Nonpareil is selected, the limit of the particle size of the produced granular preparation is about 1,020 to about 500 µm. In contrast, according to the method employed in the present invention, the water-swellable substance is subjected to extrusion granulation in a swelling state. Thus, granulation can be performed at low extrusion pressure, and by means of a screen having a mesh size of 0.3 to 0.4 mm, which is difficult to employ in conventional extrusion granulation. As a result, spherical core microparticles having a particle size of, for example, 500 to 355 µm, 355 to 250 µm, and 250 to 180 µm (i.e., standards in powder preparation and fine granule preparation) can be produced. Through employment of such core particles, timed-release pharmaceutical preparations in powder and fine-granule form, which have been difficult to produce through a conventional production method, can be produced in a simple manner. Particularly, a timed-release pharmaceutical preparation which can be easily taken can be provided. In the case of a strongly bitter drug, a drug preparation which disintegrates in the oral cavity may be provided.

The general mechanism of releasing of a drug from the pharmaceutical preparation of the present invention is as follows. When perorally administered, the pharmaceutical preparation of the present invention absorbs only a small amount of water through the film in the stomach (i.e., low pH environment), and the water-swellable substance contained in the core does not swell. Thus, no drug release occurs. Subsequently, when the preparation is transferred from the stomach to an upper portion of the small intestine (i.e, higher pH environment), water begins to permeate the film, and the water-swellable substance contained in the core gradually swells. After passage of a certain period of time, the volume of the core increases by swelling of the water-swellable substance in the core, resulting in breakage of the film. As a result, the entirety of the drug is immediately released. The time required until release of the drug is defined as lag time. The lag time may be adjusted as desired by changing the proportions of the water-insoluble polymer (particularly, Eudragit RS), plasticizer, and water-insoluble filler. Particularly, the lag time is prolonged by increasing the amount of plasticizer, and a short lag time and rapid drug release after passage of the lag time can be attained by increasing the amount of water-insoluble filler. These parameters can be also modified by changing the thickness of the film. Therefore, according to the present invention, through adjusting the time ranging from the discharge of the drug from the stomach to reaching the drug action site or drug absorption site (i.e., lag time), there can be provided a pH-dependent timed-release pharmaceutical preparation which immediately releases the drug at a lower gastrointestinal tract portion of interest.

More specifically, a plasticizer is added to the core containing a drug and a water-swellable substance in an amount of 2 mass% or more with respect to the total mass (solid content) of the water-insoluble polymer. The film is formed from a composition containing a water-insoluble filler added thereto in an amount of 30 mass% or more with respect to the total mass of the film. From these materials, there can be more reliably produced a timed-release pharmaceutical preparation which has a lag time of 5 minutes or shorter, 10 minutes or shorter, or 15 minutes or shorter and which releases 80 mass% or more of the drug within 12 minutes, 15 minutes, or 20 minutes (preferably within 5 minutes, 10 minutes, or 15 minutes) after passage of the lag time. Furthermore, a plasticizer is used in an amount of 7 mass% or more with respect to the total mass (solid content) of the water-insoluble polymer, and the film is formed from a composition containing a water-insoluble filler added thereto in an amount of 30 mass% or more with respect to the total mass of the film. In this case, there can be more reliably produced a timed-release pharmaceutical preparation which does not release the drug in the acidic and neutral pH regions and which releases the drug only in the alkaline pH region after passage of a certain period of lag time. Therefore, a characteristic feature of the pharmaceutical preparation of the present invention resides in that, after peroral administration, the preparation does not release the drug by gastric juice and immediately releases the drug after passage of a certain period after discharge from the stomach, to thereby attain a sufficiently effective blood drug level. In other words, through modifying the amount of triethyl citrate in the coated preparation or the coating amount, the drug release initiation time after discharge from the stomach can be controlled as desired.
Thus, after discharge from the stomach, pulse-like immediate drug release after passage of a certain period can be realized, and the drug release can be realized at a specific site of the intestine. Therefore, the drug can be caused to act on a local site of the large intestine or other sites, and the drug can be delivered to the large intestine with preventing degradation of peptide, protein etc. Examples

The present invention will next be described in detail by way of Examples and Comparative Examples, which should not be construed as limiting the invention thereto. Hereinafter, the unit "%" means "mass%," and "lag time" and "T_{80%}" in the Tables are on the minute basis.

### Example 1

Theophylline (product of Shiratori Pharmaceutical Co., Ltd.) (100 g) and low-substituted hydroxypropyl cellulose (L-HPC LH31, product of Shin-Etsu Chemical Co., Ltd.) (900 g) were mixed by means of a vertical granulator FM-VG-25 (product of Powrex Corporation), and the mixture was kneaded with 10-mass% aqueous ethanol solution (2,900 g). The kneaded product was subjected to extrusion granulation by means of Twin Dome Granulator TDG-80 (product of Fuji Paudal Co., Ltd.) equipped with a screen having a mesh size of 0.8 mm, and the granules were formed into spherical particles by means of Marumerizer Q400 (product of Fuji Paudal Co., Ltd.). Thereafter, the particles were dried by means of a fluidized-bed dryer (Model WSG-55, product of Okawara Corporation) and sieved by means of a 20-mesh sieve and a 30-mesh sieve, to thereby produce spherical granules containing 10% theophylline and having a particle size of 20- to 30-mesh (840 to 500 µm).
Subsequently, a coating liquid (2,000 g or 2,500 g) shown in Table 1 was sprayed to the spherical granules (500 g) by means of a tumbling-fluidized-bed coating apparatus MP-01 (product of Powrex Corporation), to thereby produce a granular preparation coated with the coating liquid in a coating amount (solid content) of 40% or 50%.

**[Table 1]**

| | |
|---|---|
| Coating liquid formulation | |

| Ingredients | parts by mass |
|---|---|
| Eudragit RS30D | 15.3 (solid content: 4.6) |
| Triethyl citrate (TEC) | 0.4 |
| Talc SK-C | 5 |
| Purified water | 79.3 |
| Total | 100 |

### Example 2

In a manner similar to that of Example 1, a coating liquid (2,000 g or 25,000 g) shown in Table 2 was sprayed to the 10% theophylline-containing granules (20- to 30-mesh) (500 g) produced in Example 1, to thereby produce a granular preparation coated with the coating liquid in a coating amount (solid content) of 40% or 50%.

**[Table 2]**

| | |
|---|---|
| Coating liquid formulation | |

| Ingredients | parts by mass |
|---|---|
| Eudragit RS30D | 15 (solid content: 4.5) |
| Triethyl citrate (TEC) | 0.5 |
| Talc SK-C | 5 |
| Purified water | 79.5 |
| Total | 100 |

### Example 3

In a manner similar to that of Example 1, a coating liquid (2,000 g or 2,500 g) shown in Table 3 was sprayed to the 10% theophylline-containing granules (20- to 30-mesh) (500 g) produced in Example 1, to thereby produce a granular preparation coated with the coating liquid in a coating amount (solid content) of 40% or 50%.

**[Table 3]**

| | |
|---|---|
| Coating liquid formulation | |

| Ingredients | parts by mass |
|---|---|
| Eudragit RS30D | 13.3 (solid content: 4.0) |
| Triethyl citrate (TEC) | 1 |
| Talc SK-C | 5 |
| Purified water | 80.7 |
| Total | 100 |

### Example 4

A coating liquid (2,000 g or 3,000 g) shown in Table 4 was sprayed to the 10% theophylline-containing granules (20-to 30-mesh) (500 g) produced in Example 1, to thereby produce a granular preparation coated with the coating liquid in a coating amount (solid content) of 40% or 60%.

**[Table 4]**

| | |
|---|---|
| Coating liquid formulation | |

| Ingredients | parts by mass |
|---|---|
| Eudragit RS30D | 16 (solid content: 4.8) |
| Triethyl citrate (TEC) | 0.2 |
| Talc SK-C | 5 |
| Purified water | 78.8 |
| Total | 100 |

### Example 5

A coating liquid (2,000 g or 3,000 g) shown in Table 5 was sprayed to the 10% theophylline-containing granules (20-to 30-mesh) (500 g) produced in Example 1, to thereby produce a granular preparation coated with the coating liquid in a coating amount (solid content) of 40% or 60%.

**[Table 5]**

| | |
|---|---|
| Coating liquid formulation | |

| Ingredients | parts by mass |
|---|---|
| Eudragit RS30D | 15.7 (solid content: 4.7) |
| Triethyl citrate (TEC) | 0.3 |
| Talc SK-C | 5 |
| Purified water | 79 |
| Total | 100 |

### Test Example 1

The pharmaceutical preparations produced in Examples 1 to 5 were subjected to a dissolution test of the following test method.

### Dissolution test method: Dissolution test (2) 2nd method (paddle method), Japanese Pharmacopoeia 15th edition Test liquid:

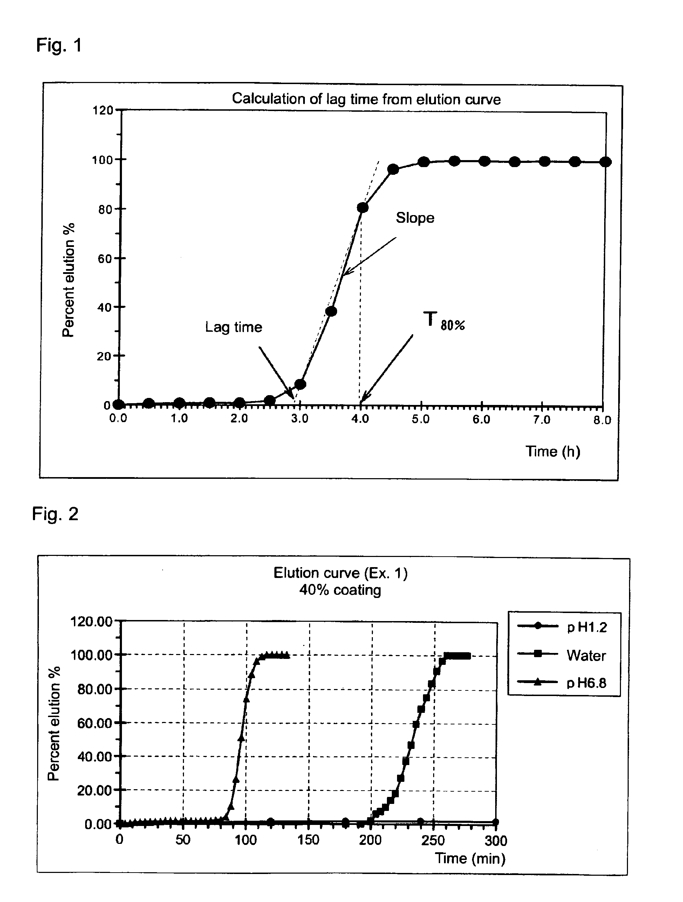

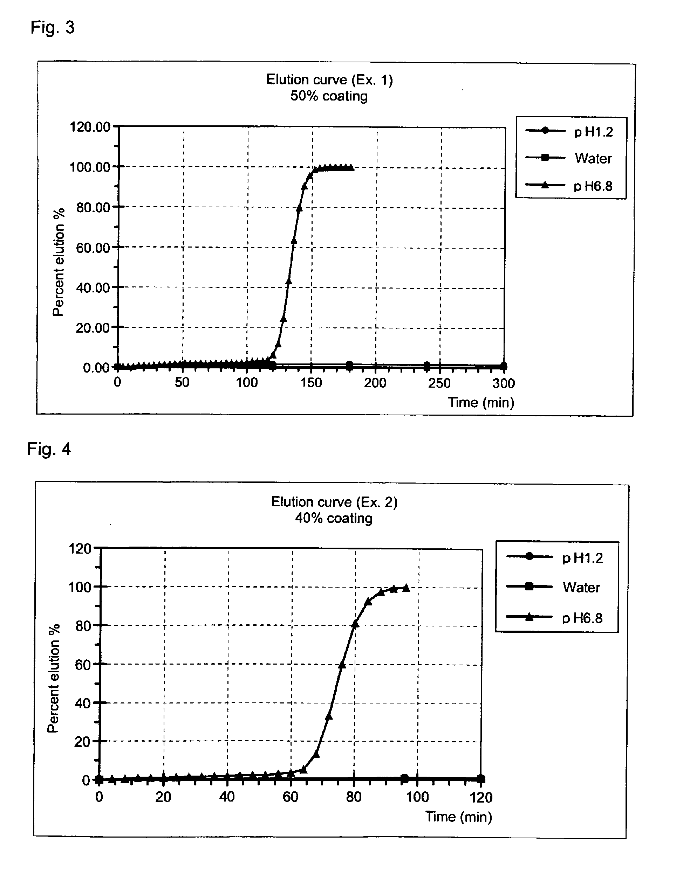

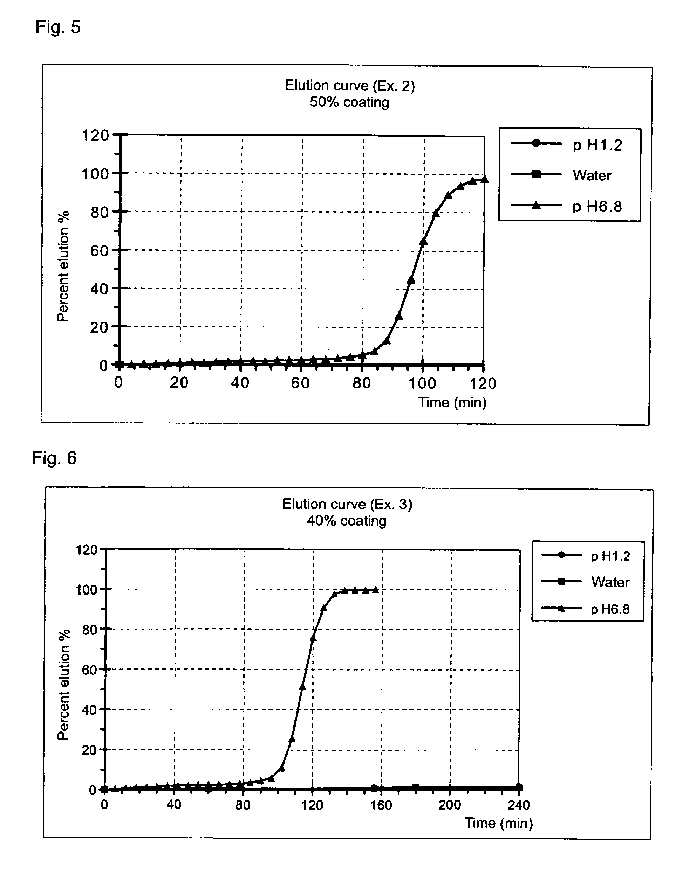

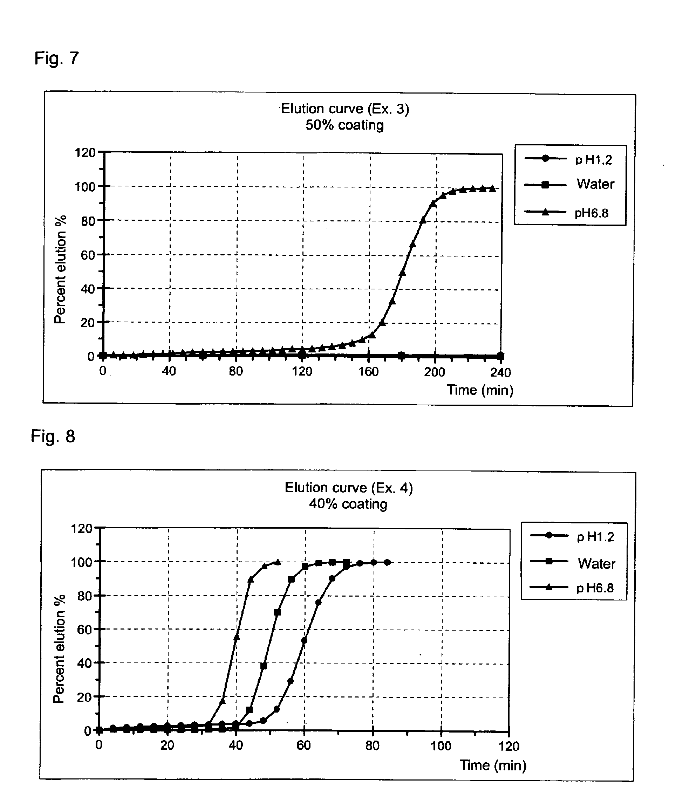

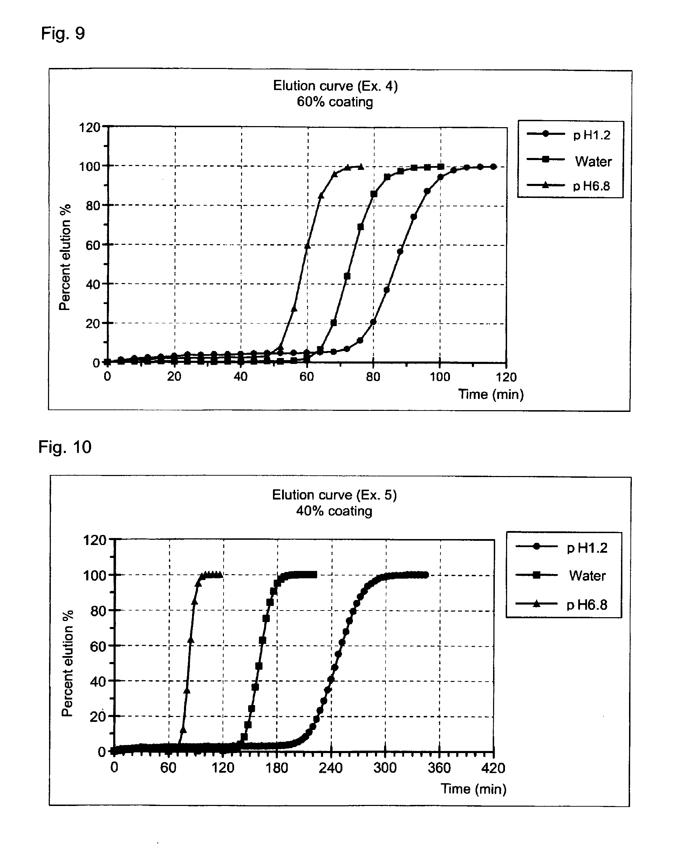

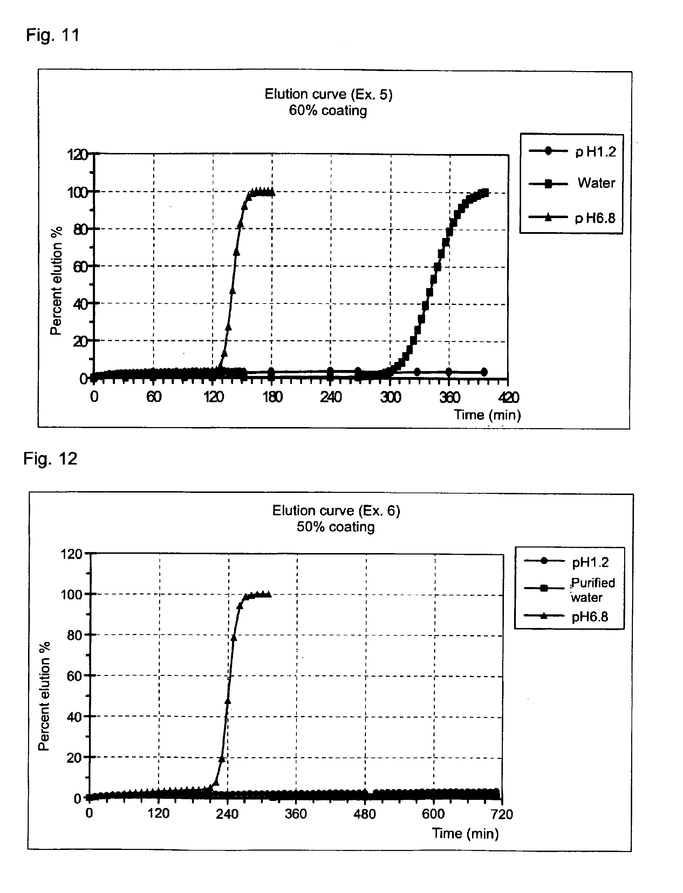

A pharmaceutical preparation was added to water (900 mL) in such an amount that the theophylline content was adjusted to 60 mg, and the mixture was stirred at 100 rpm/minute. The amount of eluted theophylline was measured through the UV method (wavelength: 267 nm). The percentage of the elution amount to the amount of theophylline incorporated to the preparation was calculated and evaluated. Figs. 2 and 3 (Example 1), Figs. 4 and 5 (Example 2), Figs. 6 and 7 (Example 3), Figs. 8 and 9 (Example 4), and Figs. 10 and 11 (Example 5) show elution curves.

For each preparation, an elution curve as shown in Fig. 1 was drawn. At the elution amounts of 20 to 80%, correlation analysis was performed. Specifically, correlation factor, slope of the line, lag time (duration when a drug is not released), T_{80%}, and T_{80%}-lag time (drug releasability) were calculated, wherein the lag time is indicated by the point of the line crossing the x-axis (time axis), and T_{80%} means the duration until the elution amount reaches 80%. The analytical results are shown in Table 6 (Example 1), Table 7 (Example 2), Table 8 (Example 3), Table 9 (Example 4), and Table 10 (Example 5).

**[Table 6]**

| | | | | | | |
|---|---|---|---|---|---|---|
| Characteristics of timed-release pharmaceutical preparation calculated from elution curve (Example 1) | | | | | | |

| | 40% | | | 50% | | |
|---|---|---|---|---|---|---|
| | pH 1.2 | Water | pH 6.8 | pH 1.2 | Water | pH 6.8 |
| Correlation factor | - | 0.985 | 0.988 | - | - | 0.980 |
| Slope | - | 2.006 | 4.673 | - | - | 3.561 |
| Lag time | - | 207.1 | 84.9 | - | - | 118.6 |
| T_{80%} | - | 247.0 | 102.0 | - | - | 141.1 |
| Releasability | - | 39.9 | 17.1 | - | - | 22.5 |

| | | | | | | |
|---|---|---|---|---|---|---|
| -: Not calculated due to too long lag time (the same applies to the other tables) | | | | | | |

**[Table 7]**

| | | | | | | |
|---|---|---|---|---|---|---|
| Characteristics of timed-release pharmaceutical preparation calculated from elution curve (Example 2) | | | | | | |

| | 40% | | | 50% | | |
|---|---|---|---|---|---|---|
| | pH 1.2 | Water | pH 6.8 | pH 1.2 | Water | pH 6.8 |
| Correlation factor | - | - | 0.990 | - | - | 0.983 |
| Slope | - | - | 4.9 | - | - | 3.3 |
| Lag time | - | - | 64.2 | - | - | 81.8 |
| T_{80%} | - | - | 80.6 | - | - | 105.8 |
| Releasability | - | - | 16.4 | - | - | 24.0 |

**[Table 8]**

| | | | | | | |
|---|---|---|---|---|---|---|
| Characteristics of timed-release pharmaceutical preparation calculated from elution curve (Example 3) | | | | | | |

| | 40% | | | 50% | | |
|---|---|---|---|---|---|---|
| | pH 1.2 | Water | pH 6.8 | pH 1.2 | Water | pH 6.8 |
| Correlation factor | - | - | 0.984 | - | - | 0.994 |
| Slope | - | - | 3.2 | - | - | 2.4 |
| Lag time | - | - | 97.3 | - | - | 158.6 |
| T_{80%} | - | - | 122.5 | - | - | 191.9 |
| Releasability | - | - | 25.2 | - | - | 33.4 |

**[Table 9]**

| | | | | | | |
|---|---|---|---|---|---|---|
| Characteristics of timed-release pharmaceutical preparation calculated from elution curve (Example 4) | | | | | | |

| | 40% | | | 60% | | |
|---|---|---|---|---|---|---|
| | pH 1.2 | Water | pH 6.8 | pH 1.2 | Water | pH 6.8 |
| Correlation factor | 0.989 | 0.979 | 0.985 | 0.964 | 0.977 | 0.996 |
| Slope | 4.7 | 5.8 | 7.7 | 3.1 | 4.4 | 6.6 |
| Lag time | 48.5 | 41.4 | 32.6 | 68.9 | 61.3 | 51.2 |
| T_{80%} | 65.6 | 55.2 | 43.0 | 95.1 | 79.5 | 63.3 |
| Releasability | 17.2 | 13.8 | 10.4 | 26.2 | 18.1 | 12.1 |

**[Table 10]**

| | | | | | | |
|---|---|---|---|---|---|---|
| Characteristics of timed-release pharmaceutical preparation calculated from elution curve (Example 5) | | | | | | |

| | 40% | | | 60% | | |
|---|---|---|---|---|---|---|
| | pH 1.2 | Water | pH 6.8 | pH 1.2 | Water | pH 6.8 |
| Correlation factor | 0.999 | 0.998 | 0.998 | - | 0.998 | 0.998 |
| Slope | 1.5 | 3.0 | 6.2 | - | 1.6 | 4.5 |
| Lag time | 212.4 | 143.6 | 74.0 | - | 310.7 | 129.3 |
| T_{80%} | 264.6 | 170.1 | 87.0 | - | 360.9 | 147.1 |
| Releasability | 52.2 | 26.5 | 13.0 | - | 50.2 | 17.8 |

In Examples 4 and 5, timed-release pharmaceutical preparations which release a drug after passage of the lag time were obtained. Therefore, when the plasticizer is incorporated in an amount of 7 mass% or more with respect to the total mass of the water-insoluble polymer, there may be produced a pH-dependent timed-release pharmaceutical preparation which releases a drug only in the alkaline pH region. Thus, preparations having a triethyl citrate content of 8.7% (Example 1), 11.1% (Example 2), and 25.0% (Example 3) were produced. These preparations were confirmed to be pH-dependent timed-release pharmaceutical preparations which did not release a drug in a medium of pH 1.2 and in purified water (neutral) and released the drug in a medium of pH 6.8 after passage of the lag time.

### Comparative Example 1

Nonpareil 103 (30- to 42-mesh, product of Freund Corporation) (5,000 g) was placed in a centrifugal fluidizing granulator (CF-3601). While the particles were tumbled, a solution of hydroxypropyl cellulose (HPC-L, product of Nippon Soda Co., Ltd.) (20 g) dissolved in water-ethanol (1:1) (500 g) was sprayed to the particles, and simultaneously a mixture of theophylline (product of Shiratori Pharmaceutical Co., Ltd.) (1,250 g) and lactose (730 g) was gradually added to Nonpareil so as to form coating film, to thereby yield 50% theophylline-containing spherical granules having a particle size of 20- to 30-mesh.
Subsequently, a coating liquid (2,000 g or 3,000 g) shown in Table 1 was sprayed to the spherical granules (500 g) by means of a tumbling-fluidized-bed coating apparatus MP-01 (product of Powrex Corporation), to thereby produce a granular preparation coated with the coating liquid in a coating amount (solid content) of 40% or 60%.

The thus-produced pharmaceutical preparations were subjected to the dissolution test in a manner similar to that of Test Example 1. Both 40%-coated granules and 50%-coated granules did not exhibit lag time.

### Example 6

Anhydrous caffeine (product of Neiyaku Kagaku Co., Ltd.) (500 g) and low-substituted hydroxypropyl cellulose (L-HPC LH31, product of Shin-Etsu Chemical Co., Ltd.) (500 g) were kneaded with 10% aqueous ethanol solution (1,500 g). In a manner similar to that of Example 1, 50% anhydrous-caffeine-containing spherical granules having a particle size of 20- to 30-mesh (840 to 500 µm) was produced.
Subsequently, a coating liquid (2,500 g or 3,000 g) shown in Table 1 was sprayed to the spherical granules (500 g) by means of a tumbling-fluidized-bed coating apparatus MP-01 (product of Powrex Corporation), to thereby produce a granular preparation coated with the coating liquid in a coating amount (solid content) of 50% or 60%.

### Example 7

Acetaminophen (product of Yamamoto Corporation Co., Ltd.) (300 g) and low-substituted hydroxypropyl cellulose (L-HPC LH31, product of Shin-Etsu Chemical Co., Ltd.) (700 g) were kneaded with 10% aqueous ethanol solution (2,100 g). In a manner similar to that of Example 1, 30% acetaminophen-containing spherical granules having a particle size of 20-to 30-mesh (840 to 500 µm) was produced.
Subsequently, a coating liquid (2,500 g or 3,000 g) shown in Table 1 was sprayed to the spherical granules (500 g) by means of a tumbling-fluidized-bed coating apparatus MP-01 (product of Powrex Corporation), to thereby produce a granular preparation coated with the coating liquid in a coating amount (solid content) of 50% or 60%.

### Example 8

Diphenhydramine hydrochloride (product of Kongo Chemical Co., Ltd.) (850 g) and low-substituted hydroxypropyl cellulose (L-HPC LH31, product of Shin-Etsu Chemical Co., Ltd.) (150 g) were kneaded with 10% aqueous ethanol solution (1,400 g). In a manner similar to that of Example 1, 85% anhydrous-caffeine-containing spherical granules having a particle size of 20- to 30-mesh (840 to 500 µm) was produced.
Subsequently, a coating liquid (2,500 g or 3,000 g) shown in Table 1 was sprayed to the spherical granules (500 g) by means of a tumbling-fluidized-bed coating apparatus MP-01 (product of Powrex Corporation), to thereby produce a granular preparation coated with the coating liquid in a coating amount (solid content) of 50% or 60%.

### Test Example 2

The pharmaceutical preparations produced in Examples 6 to 8 were subjected to the dissolution test in a manner similar to that of Test Example 1.
Figs. 12 to 17 show elution curves. Tables 11 to 13 show lag time values and T_{80%} values calculated from the elution curves.

**[Table 11]**

| | | | | | | |
|---|---|---|---|---|---|---|
| Characteristics of timed-release pharmaceutical preparation calculated from elution curve (Example 6) | | | | | | |

| | 50% | | | 60% | | |
|---|---|---|---|---|---|---|
| | pH 1.2 | Purified water | pH 6.8 | pH 1.2 | Purified water | pH 6.8 |
| Correlation factor | - | - | 0.990 | - | - | 0.992 |
| Slope | - | - | 2.4 | - | - | 2.1 |
| Lag time | - | - | 219.0 | - | - | 277.6 |
| T_{80%} | - | - | 252.8 | - | - | 315.4 |
| Releasability | - | - | 33.8 | - | - | 37.8 |

**[Table 12]**

| | | | | | | |
|---|---|---|---|---|---|---|
| Characteristics of timed-release pharmaceutical preparation calculated from elution curve (Example 7) | | | | | | |

| | 50% | | | 60% | | |
|---|---|---|---|---|---|---|
| | pH 1.2 | Water | pH 6.8 | pH 1.2 | Water | pH 6.8 |
| Correlation factor | - | 0.989 | 0.962 | - | - | 0.983 |
| Slope | - | 0.6 | 2.2 | - | - | 2.0 |
| Lag time | - | 572.2 | 169.8 | - | - | 215.9 |
| T_{80%} | - | 697.8 | 206.9 | - | - | 255.5 |
| Releasability | - | 125.6 | 37.1 | - | - | 39.7 |

**[Table 13]**

| | | | | | | |
|---|---|---|---|---|---|---|
| Characteristics of timed-release pharmaceutical preparation calculated from elution curve (Example 8) | | | | | | |

| | 50% | | | 60% | | |
|---|---|---|---|---|---|---|
| | pH 1.2 | Water | pH 6.8 | pH 1.2 | Water | pH 6.8 |
| Correlation factor | 0.997 | 0.998 | 0.955 | 0.998 | 0.999 | 0.948 |
| Slope | 1.505 | 1.536 | 4.495 | 1.348 | 1.326 | 2.983 |
| Lag time | 275.7 | 241.1 | 76.4 | 360.2 | 310.9 | 95.1 |
| T_{80%} | 328.9 | 293.2 | 94.2 | 419.6 | 371.3 | 122.0 |
| Releasability | 53.2 | 52.1 | 17.8 | 59.4 | 60.3 | 26.8 |

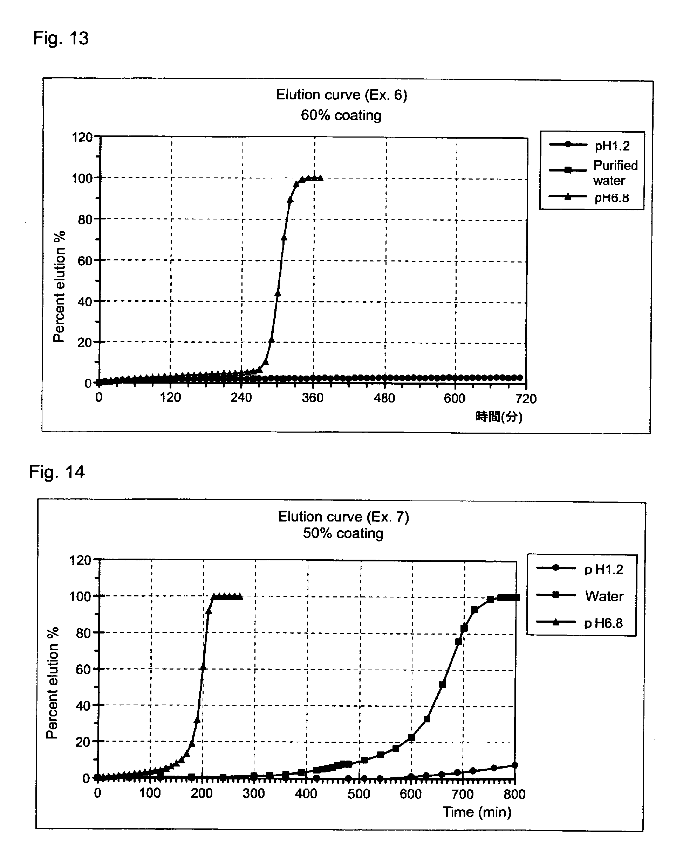

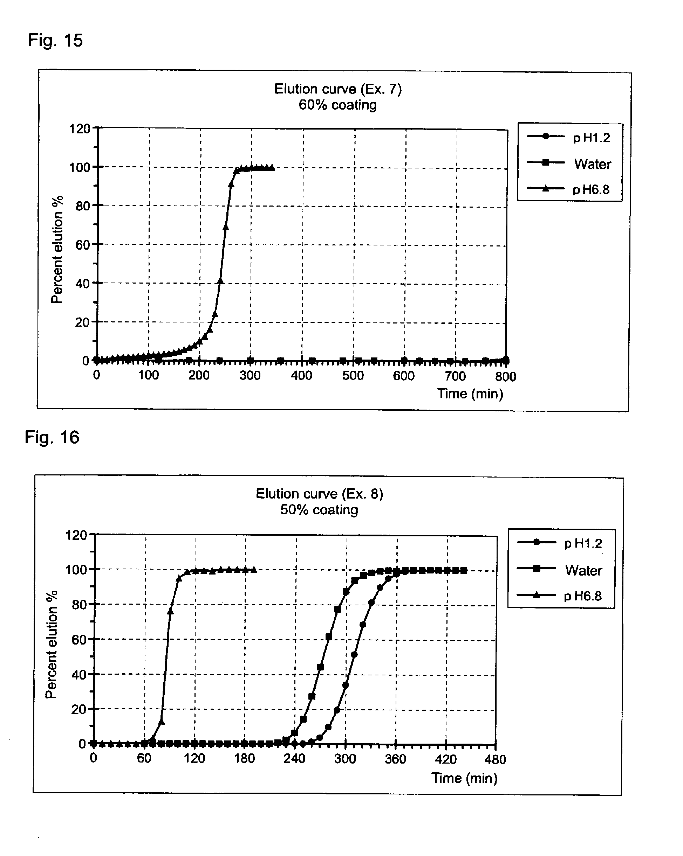

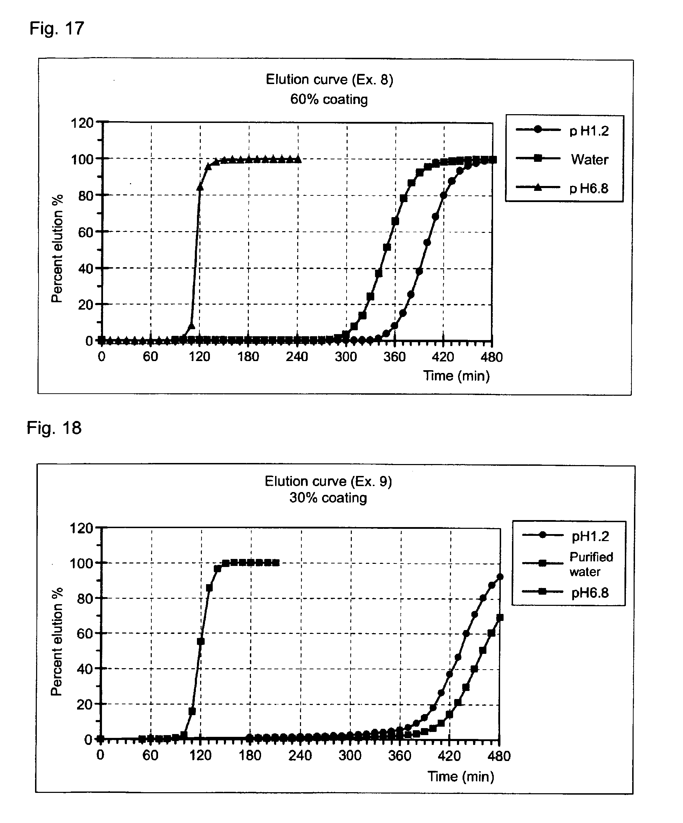

Similar to the pharmaceutical preparation of Example 1, preparations of anhydrous caffeine (Example 6, Figs. 12 and 13, Table 11), of acetaminophen (Example 7, Figs. 14 and 15, Table 12), and of diphenhydramine hydrochloride (Example 8, Figs. 16 and 17, Table 13) were found to be timed-release pharmaceutical preparations which did not release a drug in the acidic and neutral pH regions and immediately released the drug in a pulse-like manner in the alkaline pH region (6.8) after passage of a certain period of lag time.

### Example 9

In a manner similar to that of Example 1, a coating liquid (1,500 g or 2,000 g) shown in Table 14 was sprayed to the 10% theophylline-containing granules (20- to 30-mesh) (500 g) produced in Example 1, to thereby produce a granular preparation coated with the coating liquid in a coating amount (solid content) of 30% or 40%.

**[Table 14]**

| | |
|---|---|
| Coating liquid formulation | |

| Ingredients | parts by mass |
|---|---|
| Eudragit RSPO | 6.5 |
| Triethyl citrate (TEC) | 0.5 |
| Talc SK-C | 3 |
| Ethanol (Japanese Pharmacopoeia) | 90 |
| Total | 100 |

### Example 10

In a manner similar to that of Example 1, a coating liquid (1,750 g or 2,000 g) shown in Table 14 was sprayed to the 50% anhydrous-caffeine-containing granules (20- to 30-mesh) (500 g) produced in Example 6, to thereby produce a granular preparation coated with the coating liquid in a coating amount (solid content) of 35% or 40%.

### Example 11

In a manner similar to that of Example 1, a coating liquid (5,000 g or 5,500 g) shown in Table 14 was sprayed to the 30% acetaminophen-containing granules (20- to 30-mesh) (500 g) produced in Example 7, to thereby produce a granular preparation coated with the coating liquid in a coating amount (solid content) of 100% or 110%.

### Example 12

In a manner similar to that of Example 1, a coating liquid (1,500 g or 2,000 g) shown in Table 15 was sprayed to the 10% theophylline-containing granules (20- to 30-mesh) (500 g) produced in Example 1, to thereby produce a granular preparation coated with the coating liquid in a coating amount (solid content) of 30% or 40%.

**[Table 15]**

| | |
|---|---|
| Coating liquid formulation | |

| Ingredients | parts by mass |
|---|---|
| Eudragit RSPO | 4.6 |
| Myvacet 9-45T | 0.4 |
| Talc SK-C | 5 |
| Ethanol (Japanese Pharmacopoeia) | 90 |
| Total | 100 |

| | |
|---|---|
| Myvacet 9-45T: (ingredient) glycerin fatty acid ester, product of Koyo Mercantile Co., Ltd. | |

### Test Example 3

The pharmaceutical preparations produced in Examples 9 to 12 were subjected to the dissolution test in a manner similar to that of Test Example 1.
Figs. 18 to 25 show elution curves. Tables 16 to 19 show lag time values and T_{80%} values calculated from the elution curves.

**[Table 16]**

| | | | | | | |
|---|---|---|---|---|---|---|
| Characteristics of timed-release pharmaceutical preparation calculated from elution curve (Example 9) | | | | | | |

| | | 30% | | | 40% | |
|---|---|---|---|---|---|---|
| | pH 1.2 | Purified water | pH 6.8 | pH 1.2 | Purified water | pH 6.8 |
| Correlation factor | 0.996 | 0.998 | 0.997 | 0.991 | 0.996 | 0.995 |
| Slope | 1.02 | 0.91 | 3.51 | 0.84 | 0.59 | 2.83 |
| Lag time | 381.9 | 405.6 | 105.1 | 452.1 | 471.7 | 127.6 |
| T_{80%} | 460.2 | 493.2 | 127.9 | 547.3 | 607.1 | 155.9 |
| Releasability | 78.3 | 87.6 | 22.8 | 95.3 | 135.4 | 28.3 |

**[Table 17]**

| | | | | | | |
|---|---|---|---|---|---|---|
| Characteristics of timed-release pharmaceutical preparation calculated from elution curve (Example 10) | | | | | | |

| | 35% | | | 40% | | |
|---|---|---|---|---|---|---|
| | pH 1.2 | Purified water | pH 6.8 | pH 1.2 | Purified water | pH 6.8 |
| Correlation factor | 0.994 | 0.998 | 0.995 | 0.997 | 0.998 | 0.995 |
| Slope | 0.51 | 0.38 | 1.51 | 0.46 | 0.30 | 1.51 |
| Lag time | 419.1 | 547.1 | 230.8 | 509.5 | 639.7 | 270.8 |
| T_{80%} | 575.8 | 757.3 | 283.8 | 682.8 | 906.3 | 323.8 |
| Releasability | 156.8 | 210.1 | 53.0 | 173.3 | 266.6 | 53.0 |

**[Table 18]**

| | | | | | | |
|---|---|---|---|---|---|---|
| Characteristics of timed-release pharmaceutical preparation calculated from elution curve (Example 11) | | | | | | |

| | 100% | | | 110% | | |
|---|---|---|---|---|---|---|
| | pH 1.2 | Purified water | pH 6.8 | pH 1.2 | Purified water | pH 6.8 |
| Correlation factor | 0.996 | 0.989 | 0.988 | 0.992 | 0.995 | 0.995 |
| Slope | 0.36 | 0.55 | 1.97 | 0.29 | 0.43 | 2.02 |
| Lag time | 616.3 | 538.3 | 128.2 | 707.5 | 625.4 | 144.7 |
| T_{80%} | 840.5 | 682.8 | 168.7 | 978.9 | 809.4 | 184.3 |
| Releasability | 224.2 | 144.6 | 40.5 | 271.3 | 183.9 | 39.7 |

**[Table 19]**

| | | | | | | |
|---|---|---|---|---|---|---|
| Characteristics of timed-release pharmaceutical preparation calculated from elution curve (Example 12) | | | | | | |

| | 30% | | | 40% | | |
|---|---|---|---|---|---|---|
| | pH 1.2 | Purified water | pH 6.8 | pH 1.2 | Purified water | pH 6.8 |
| Correlation factor | 0.994 | - | 0.994 | - | - | 0.984 |
| Slope | 0.41 | - | 1.07 | - | - | 0.66 |
| Lag time | 341.7 | - | 211.3 | - | - | 259.0 |
| T_{80%} | 536.9 | - | 286.3 | - | - | 381.0 |
| Releasability | 195.1 | - | 75.0 | - | - | 122.0 |

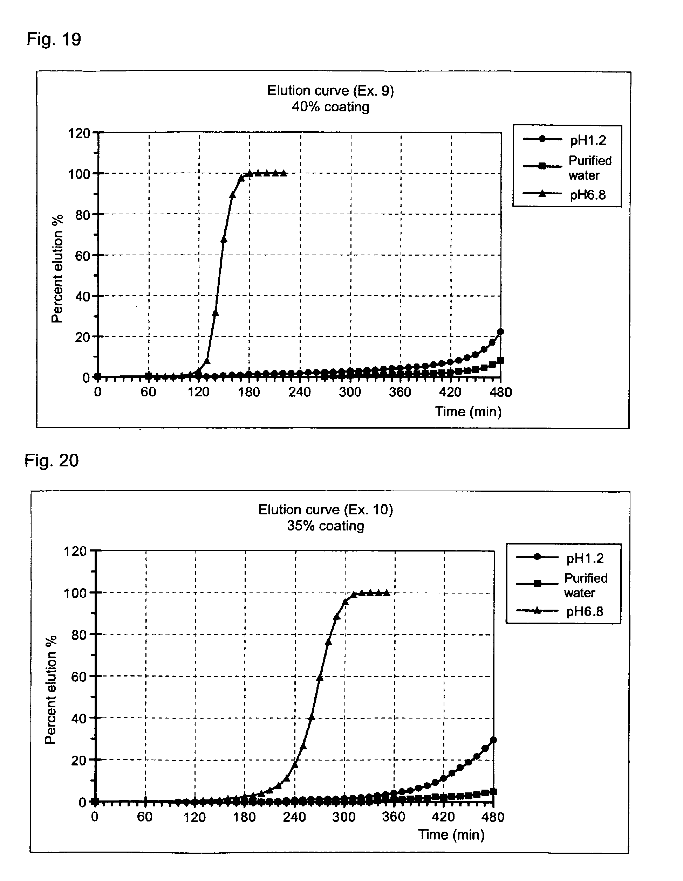

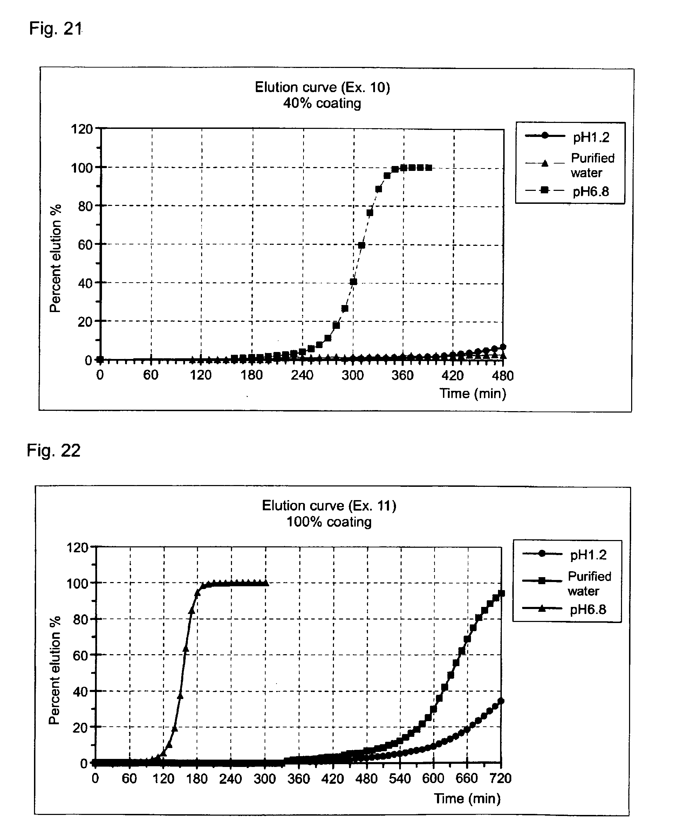

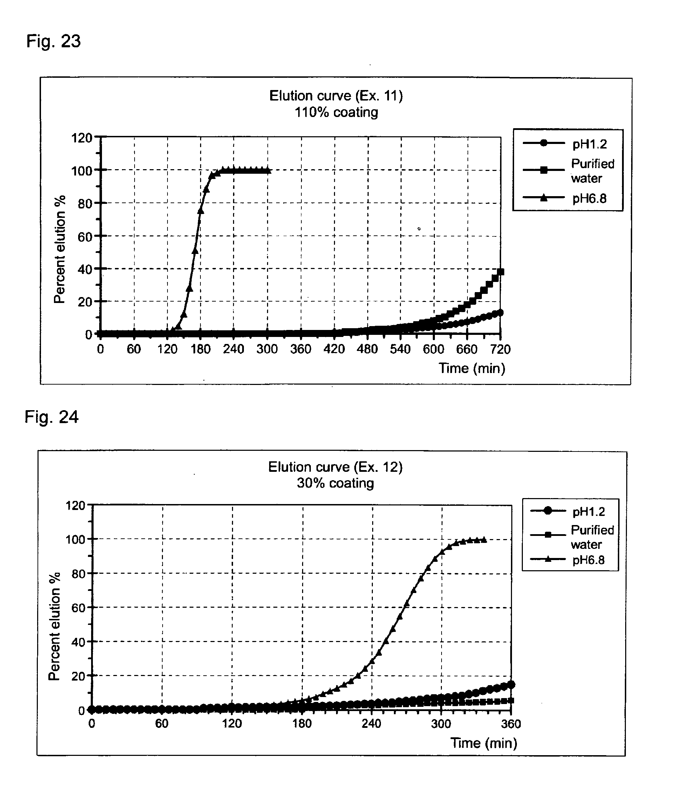

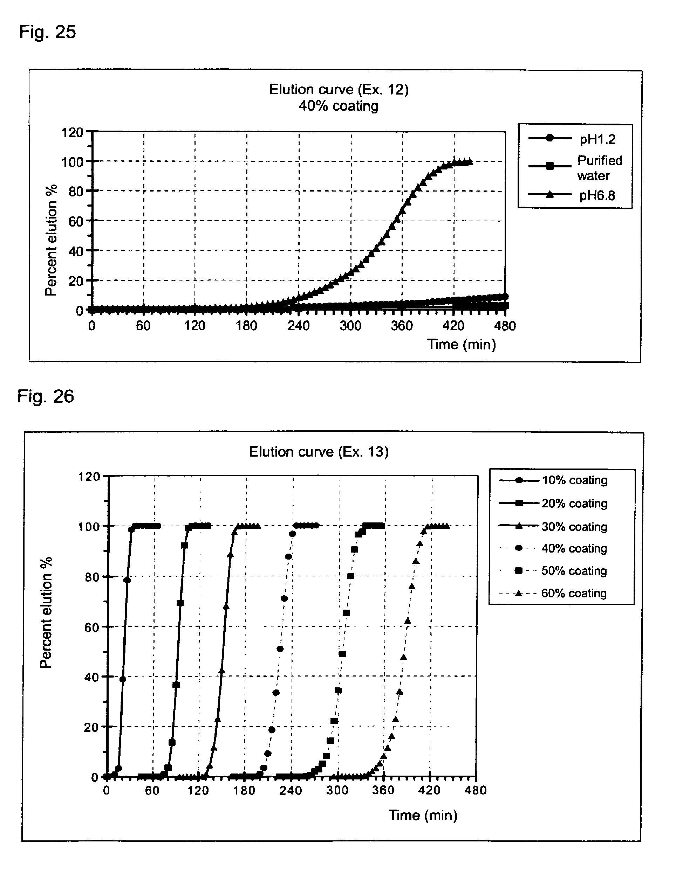

In the cases where Eudragit RS30D (aqueous) was changed to Eudragit RSPO (organic solvent-based), and drugs including theophylline (Example 9, Figs. 18 and 19, Table 16), anhydrous caffeine (Example 10, Figs. 20 and 21, Table 17), and acetaminophen (Example 11, Figs. 22 and 23, Table 18) were used, there were produced timed-release pharmaceutical preparations which did not release a drug in the acidic and neutral pH regions and rapidly and immediately released the drug in the alkaline pH region (6.8) after passage of a certain period of lag time, similar to Examples 1, 6, and 7.
In the case of Example 12 (Fig. 24 and 25, Table 19) in which glycerin fatty acid ester was used instead of triethyl citrate, there were produced timed-release pharmaceutical preparations which did not release a drug in the acidic and neutral pH regions and rapidly and immediately released the drug in the alkaline pH region (6.8) after passage of a certain period of lag time, similar to Example 1.

### Example 13

Diphenhydramine hydrochloride (product of Kongo Chemical Co., Ltd.) (300 g) and low-substituted hydroxypropyl cellulose (L-HPC LH31, product of Shin-Etsu Chemical Co., Ltd.) (700 g) were mixed by means of a vertical granulator FM-VG-25 (product of Powrex Corporation), and the mixture was kneaded with 10% aqueous ethanol solution (2,300 g). The kneaded product was processed in a manner similar to that of Example 1, to thereby produce spherical granules containing 30% diphenhydramine hydrochloride and having a particle size of 20- to 30-mesh.
Subsequently, a coating liquid (500 g, 1,000 g, 1,500 g, 2,000, 2,500, or 3,000 g) shown in Table 20 was sprayed to the spherical granules (500 g) by means of a fluidized-bed coating apparatus MP-01 (tumbling-fluidized-bed coating apparatus) (product of Powrex Corporation), to thereby produce a granular preparation coated with the coating liquid in a coating amount (solid content) of 10%, 20%, 30%, 40%, 50%, or 60%.

**[Table 20]**

| | |
|---|---|
| Coating liquid formulation | |

| Ingredients | mass% |
|---|---|
| Eudragit RS30D (solid content: 30 mass%) | 15.8 (solid content: 4.74) |
| Triethyl citrate | 0.26 |
| Talc SK-C | 5.00 |
| Purified water | 78.94 |
| Total | 100.00 |

### Test Example 4

The pharmaceutical preparation produced in Example 13 was subjected to the dissolution test in a manner similar to that of Test Example 1.
Fig. 26 shows elution curves. Table 21 shows lag time values and T_{80%} values calculated from the elution curves.

**[Table 21]**

| | | | | | | |
|---|---|---|---|---|---|---|
| Characteristics of timed-release pharmaceutical preparation calculated from elution curve (Example 13) | | | | | | |

| | Coating amount (%) | | | | | |
|---|---|---|---|---|---|---|
| | 10% | 20% | 30% | 40% | 50% | 60% |
| Correlation factor | 0.983 | 0.997 | 0.992 | 0.995 | 0.994 | 0.990 |
| Slope | 6.179 | 5.398 | 4.048 | 3.278 | 2.731 | 2.316 |
| Lag time | 13.4 | 82.7 | 138.5 | 208.8 | 286.4 | 363.3 |
| T_{80%} | 26.3 | 97.5 | 158.2 | 233.2 | 315.7 | 397.8 |
| Releasability | 12.9 | 14.8 | 19.8 | 24.4 | 29.3 | 34.5 |

In Example 13, the lag time was found to be controlled by modifying the amount of film coating.

### Example 14

Diphenhydramine hydrochloride (product of Kongo Chemical Co., Ltd.) (500 g) and low-substituted hydroxypropyl cellulose (L-HPC LH31, product of Shin-Etsu Chemical Co., Ltd.) (500 g) were mixed by means of a vertical granulator FM-VG-25 (product of Powrex Corporation), and the mixture was kneaded with 10% aqueous ethanol solution (1,800 g). The kneaded product was subjected to extrusion granulation by means of Twin Dome Granulator TDG-80 (product of Fuji Paudal Co., Ltd.) equipped with a 0.8-mm screen, and the granules were rendered to be spherical by means of Marumerizer Q400 (product of Fuji Paudal Co., Ltd.), to thereby produce spherical granules. The granules were dried by means of a fluidized-bed drier (model WSG-5, product of Okawara Corporation), and the particle size thereof was adjusted by means of a 30-mesh (500 µm) sieve and a 40-mesh (420 µm) sieve, to thereby produce 50% diphenhydramine-hydrochloride-containing spherical granules having a particle size of 30-to 40-mesh (500 to 420 µm)
Subsequently, a coating liquid (2,000 g, 2,750 g, or 3,250 g) shown in Table 22 was sprayed to the spherical granules (500 g) by means of a fluidized-bed coating apparatus MP-01 (tumbling-fluidized-bed coating apparatus) (product of Powrex Corporation), to thereby produce a granular preparation coated with the coating liquid in a coating amount (solid content) of 40%, 55%, or 65%.

**[Table 22]**

| | |
|---|---|
| Coating liquid formulation | |

| Ingredients | mass% |
|---|---|
| Eudragit RSPO | 2.94 |
| Eudragit RLPO | 1.96 |
| Talc SK-C | 5.0 |
| Glycerin fatty acid ester | 0.1 |
| (Myvacet 9-45T) | |
| Ethanol (Japanese Pharmacopoeia) | 90 |
| Total | 100 |

### Example 15

In a manner similar to that of Example 1, a coating liquid (2,250 g or 3,000 g) shown in Table 23 was sprayed to the 50% diphenhydramine-hydrochloride-containing spherical granules (30- to 40-mesh) (500 g) produced in Example 14, to thereby produce a granular preparation coated with the coating liquid in a coating amount (solid content) of 45% or 60%.

**[Table 23]**

| | |
|---|---|
| Coating liquid formulation | |

| Ingredients | mass% |
|---|---|
| Eudragit RSPO | 3.3 |
| Eudragit RLPO | 3.3 |
| Talc SK-C | 4.0 |
| Triethyl citrate | 0.4 |
| Ethanol (Japanese Pharmacopoeia) | 90 |
| Total | 100 |

### Example 16

In a manner similar to that of Example 1, a coating liquid (2,250 g, 3,750 g, or 5,000 g) shown in Table 24 was sprayed to the 50% diphenhydramine-hydrochloride-containing spherical granules (30- to 40-mesh) (500 g) produced in Example 14, to thereby produce a granular preparation coated with the coating liquid in a coating amount (solid content) of 45%, 65%, or 100%.

**[Table 24]**

| | |
|---|---|
| Coating liquid formulation | |

| Ingredients | mass% |
|---|---|
| Eudragit RSPO | 2.35 |
| Eudragit RLPO | 2.35 |
| Glycerin fatty acid ester | 0.3 |
| (Myvacet 9-4T) | |
| Talc SK-C | 5 |
| Purified water | 63 |
| Ethanol (Japanese Pharmacopoeia) | 27 |
| Total | 100 |

### Test Example 5

The pharmaceutical preparations produced in Examples 14 to 16 were subjected to the dissolution test in a manner similar to that of Test Example 1.

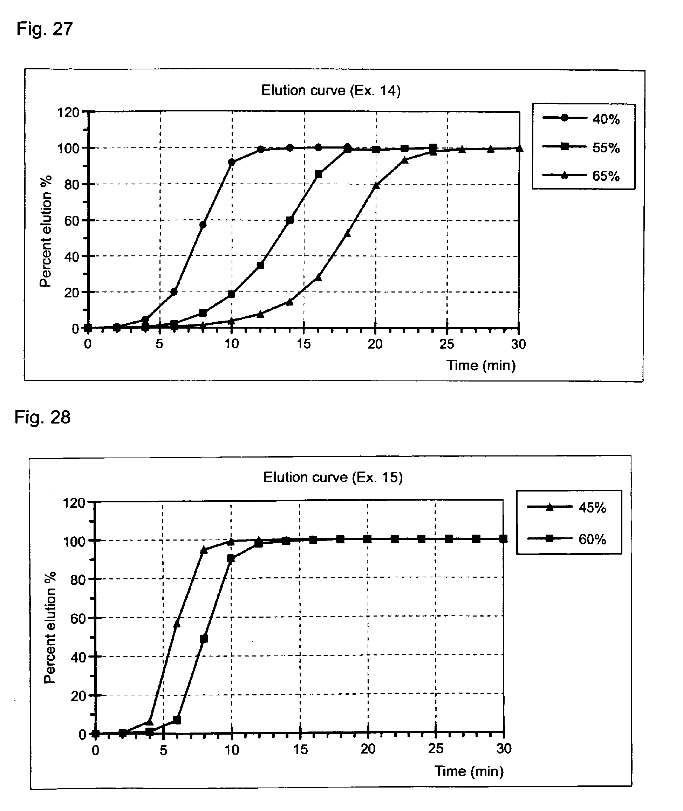

Figs. 27 to 29 show elution curves. Tables 25 to 27 show lag time values and T_{80%} values calculated from the elution curves.

**[Table 25]**

| | | | |
|---|---|---|---|
| Characteristics of timed-release pharmaceutical preparation calculated from elution curve (Example 14) | | | |

| | Coating amount | | |
|---|---|---|---|
| | 40% | 55% | 65% |
| Correlation factor | 1.000 | 0.995 | 0.997 |
| Slope | 18.1 | 11.4 | 10.5 |
| Lag time | 4.9 | 8.6 | 11.2 |
| T_{80%} | 9.3 | 15.7 | 18.8 |
| Releasability | 4.4 | 7.0 | 7.6 |

**[Table 26]**

| | | |
|---|---|---|
| Characteristics of timed-release pharmaceutical preparation calculated from elution curve (Example 15) | | |

| | Coating amount | |
|---|---|---|
| | 45% | 60% |
| Correlation factor | 0.997 | 1.000 |
| Slope | 22.3 | 20.8 |
| Lag time | 3.6 | 5.7 |
| T_{80%} | 7.2 | 9.5 |
| Releasability | 3.6 | 3.8 |

**[Table 27]**

| | | | |
|---|---|---|---|
| Characteristics of timed-release pharmaceutical preparation calculated from elution curve (Example 16) | | | |

| | Coating amount | | |
|---|---|---|---|
| | 45% | 65% | 100% |
| Correlation factor | 0.951 | 0.983 | 0.988 |
| Slope | 26.1 | 16.6 | 15.3 |
| Lag time | 2.5 | 5.8 | 9.8 |
| T_{80%} | 5.5 | 10.7 | 15.0 |
| Releasability | 3.1 | 4.8 | 5.2 |

As shown in Figs. 27 to 29, the granular preparations containing 50% theophylline (particle size: 840 to 500 µm) were found to have a lag time of ≤5 minutes, ≤10 minutes, and ≤15 minutes, and release 80% or more of theophylline contained in each preparation ≤5 minutes, ≤10 minutes, and ≤15 minutes after passage of the corresponding lag time. Thus, these granular preparations were found to be useful as masked timed-release pharmaceutical preparations.

### Example 17

Diphenhydramine hydrochloride (product of Kongo Chemical Co., Ltd.) (500 g) and low-substituted hydroxypropyl cellulose (L-HPC LH31, product of Shin-Etsu Chemical Co., Ltd.) (500 g) were mixed by means of a vertical granulator FM-VG-25 (product of Powrex Corporation), and the mixture was kneaded with 10% aqueous ethanol solution (1,500 g). The kneaded product was subjected to extrusion granulation by means of Twin Dome Granulator TDG-80 (product of Fuji Paudal Co., Ltd.) equipped with a 0.5-mm screen, and the granules were rendered to be spherical by means of Marumerizer Q400 (product of Fuji Paudal Co., Ltd.), to thereby produce spherical granules. The granules were dried by means of a fluidized-bed drier (model WSG-5, product of Okawara Corporation), and the particle size thereof was adjusted by means of a 30-mesh (500 µm) sieve and a 42-mesh (355 µm) sieve, to thereby produce 50% diphenhydramine-hydrochloride-containing spherical granules having a particle size of 30-to 42-mesh (500 to 355 µm).
Subsequently, in a manner similar to that of Example 1, a coating liquid (1,000 g, 1,250 g, or 1,500 g) shown in Table 28 was sprayed to the spherical granules (500 g) by means of a tumbling-fluidized-bed coating apparatus MP-01 (product of Powrex Corporation), to thereby produce a granular preparation coated with the coating liquid in a coating amount (solid content) of 20%, 25%, or 30%.

**[Table 28]**

| | |
|---|---|
| Coating liquid formulation | |

| Ingredients | mass% |
|---|---|
| Eudragit RSPO | 3.92 |
| Glycerin fatty acid ester (9-45T) | 0.08 |
| Talc SK-C | 6 |
| Purified water | 63 |
| Ethanol (Japanese Pharmacopoeia) | 27 |
| Total | 100 |

### Example 18

In a manner similar to that of Example 1, a coating liquid (1,500 g or 1,750 g) shown in Table 29 was sprayed to the 50% diphenhydramine-hydrochloride-containing spherical granules (30- to 42-mesh, 500 to 355 µm) (500 g) produced in Example 16 by means of a tumbling-fluidized-bed coating apparatus MP-01 (product of Powrex Corporation), to thereby produce a granular preparation coated with the coating liquid in a coating amount (solid content) of 30% or 35%.

**[Table 29]**

| | |
|---|---|
| Coating liquid formulation | |

| Ingredients | mass% |
|---|---|
| Eudragit RSPO | 2.92 |
| Triethyl citrate | 0.08 |
| Talc SK-C | 7 |
| Purified water | 63 |
| Ethanol (Japanese Pharmacopoeia) | 27 |
| Total | 100 |

### Test Example 6

The pharmaceutical preparations produced in Examples 17 and 18 were subjected to the dissolution test in a manner similar to that of Test Example 1.

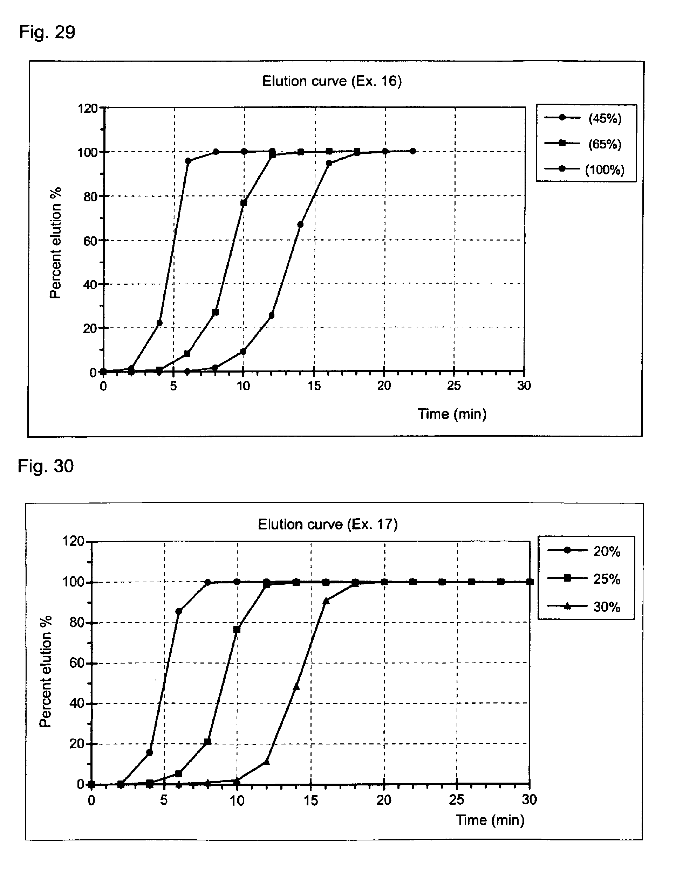

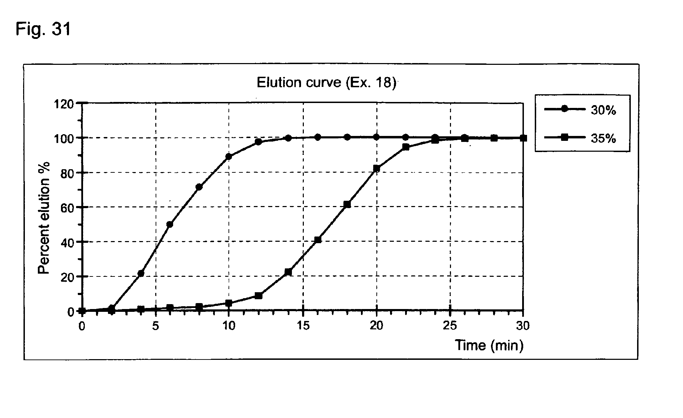

Figs. 30 and 31 show elution curves. Tables 30 and 31 show lag time values and T_{80%} values calculated from the elution curves.

**[Table 30]**

| | | | |
|---|---|---|---|
| Characteristics of timed-release pharmaceutical preparation calculated from elution curve (Example 17) | | | |

| | Coating amount (%) | | |
|---|---|---|---|
| | 20% | 25% | 30% |
| Correlation factor | 0.939 | 0.952 | 0.999 |
| Slope | 24.2 | 19.7 | 19.9 |
| Lag time | 2.6 | 6.3 | 11.5 |
| T_{80%} | 5.9 | 10.3 | 15.5 |
| Releasability | 3.3 | 4.1 | 4.0 |

**[Table 31]**

| | | |
|---|---|---|
| Characteristics of timed-release pharmaceutical preparation calculated from elution curve (Example 18) | | |

| | Coating amount (%) | |
|---|---|---|
| | 30% | 35% |
| Correlation factor | 0.994 | 0.997 |
| Slope | 11.4 | 9.4 |
| Lag time | 1.9 | 11.4 |
| T_{80%} | 9.0 | 20.0 |
| Releasability | 7.0 | 8.5 |

As shown in Figs. 30 and 31, the granular preparations (core containing 50% diphenhydramine hydrochloride and having a particle size of 500 µm to 355 µm, with talc (60% in Example 17 or 70% in Example 18)) were found to be timed-release pharmaceutical preparations which have a lag time of ≤5 minutes, ≤10 minutes, and ≤15 minutes, and release 80% or more of diphenhydramine hydrochloride contained in each preparation ≤12 minutes, ≤15 minutes, and ≤20 minutes after passage of the corresponding lag time. Thus, these granular preparations were found to be useful as masked timed-release pharmaceutical preparations.

## Claims

1. A timed-release pharmaceutical preparation, comprising a core containing a drug and a water-swellable substance, and a film containing a water-insoluble polymer, a plasticizer, and a water-insoluble filler, the core being coated with the film.

2. A timed-release pharmaceutical preparation according to claim 1, wherein the water-insoluble polymer is a (meth)acrylic water-insoluble polymer.

3. A timed-release pharmaceutical preparation according to claim 2, wherein the (meth)acrylic water-insoluble polymer is ethyl acrylate-methyl methacrylate-trimethylammoniumethyl methacrylate chloride terpolymer.

4. A timed-release pharmaceutical preparation according to any one of claims 1 to 3, wherein the plasticizer is at least one species selected from among triethyl citrate, a glycerin fatty acid ester, and triacetin.

5. A timed-release pharmaceutical preparation according to any one of claims 1 to 4, wherein the water-insoluble filler is at least one species selected from among talc, magnesium stearate, calcium stearate, kaolin, titanium oxide, magnesium oxide, calcium carbonate, magnesium carbonate, calcium phosphate, calcium sulfate, and aluminum hydroxide dry gel.

6. A timed-release pharmaceutical preparation according to any one of claims 1 to 5, wherein the water-swellable substance is at least one species selected from among low-substituted hydroxypropyl cellulose, carmellose or a salt thereof, croscarmellose sodium, carboxymethylstarch sodium, crospolyvinylpyrrolidone, crystalline cellulose, and crystalline cellulose/carmellose sodium.

7. A timed-release pharmaceutical preparation according to any one of claims 1 to 6, wherein the core is produced through wet granulation by use of water or aqueous alcohol.

8. A timed-release pharmaceutical preparation according to any one of claims 1 to 7, wherein the core has a water-swellable substance content of 15 mass% or more.

9. A timed-release pharmaceutical preparation according to any one of claims 1 to 8, wherein the film has a water-insoluble polymer content of 30 mass% or more.

10. A timed-release pharmaceutical preparation according to any one of claims 1 to 9, wherein the film has a plasticizer content, with respect to the total mass (solid content) of the water-insoluble polymer, of 2 mass% or more.

11. A timed-release pharmaceutical preparation according to any one of claims 1 to 10, wherein the film has a water-insoluble filler content of 15 mass% or more.

12. A timed-release pharmaceutical preparation according to any one of claims 1 to 11, wherein the coating amount of the film, with respect to the total mass of the core, is 10 mass% or more.

13. A timed-release pharmaceutical preparation according to any one of claims 1 to 12, which is a pH-dependent type preparation.
